# FASCICULE DE BREVET EUROPEEN

(11) **EP 1 755 620 B1**
(45) Date de publication et mention de la délivrance du brevet: **25.05.2011**
(21) Numéro de dépôt: 05775288.3
(22) Date de dépôt: 06.06.2005
(51) Int. Cl.: A61K 31/737, A61P 19/02, A61P 9/10

(54) **DERIVES DEPOLYMERISES SULFATES D'EXOPOLYSACCHARIDES (EPS), PREPARATION ET LEURS UTILISATIONS**
SULFIERTE DEPOLYMERISIERTE EXOPOLYSACCHARIDDERIVATE, IHRE HERSTELLUNG UND VERWENDUNGEN
SULPHATED DEPOLYMERISED EXOPOLYSACCHARIDE DERIVATIVES, THEIR PREPARATION AND USES

(30) Priorité: 14.06.2004 FR 0406405
(43) Date de publication de la demande: 28.02.2007
(73) Titulaire: Institut Francais de Recherche pour l'Exploitation de la Mer (IFREMER), 92130 Issy-les-Moulineaux Cedex (FR); Université René Descartes - Paris 5, 75270 Paris Cedex 06 (FR)
(72) Inventeur: MATOU, Sabine, F-93370 Montfermeil (FR); COLLIEC-JOUAULT, Sylvia, F-44100 Nantes (FR); HELLEY, Dominique, F-75004 Paris (FR); RATISKOL, Jacqueline, F-44980 Sainte Luce sur Loire (FR); SINQUIN, Corinne, F-44300 Nantes (FR); BOISSET, Claire, F-29680 Roscoff (FR); GUEZENNEC, Jean, F-29280 Plouzane (FR); FISCHER, Anne-Marie, F-75013 Paris (FR)
(74) Mandataire: Cabinet Plasseraud
(86) Numéro de dépôt international: PCT/FR2005/001378
(87) Numéro de publication internationale: WO 2006/003289

(56) Documents cités:
- WO-A-01/15654
- WO-A-99/67411
- WO-A-03/106506
- FR-A- 2 701 488
- FR-A- 2 755 142
- DATABASE WPI Section Ch, Week 199843 Derwent Publications Ltd., London, GB; Class B04, AN 1998-501676 XP002368826 & JP 10 218902 A (SEIKAGAKU KOGYO CO LTD) 18 août 1998 (1998-08-18)
- COLLIEC JOUAULT A S ET AL: "Characterization, chemical modifications and in vitro anticoagulant properties of an exopolysaccharide produced by Alteromonas infernus" BBA - GENERAL SUBJECTS, ELSEVIER SCIENCE PUBLISHERS, NL, vol. 1528, no. 2-3, 3 octobre 2001 (2001-10-03), pages 141-151, XP004312011 ISSN: 0304-4165
- GUEZENNEC J ET AL: "Sulfation and depolymerization of a bacterial exopolysaccharide of hydrothermal origin" CARBOHYDRATE POLYMERS, APPLIED SCIENCE PUBLISHERS, LTD. BARKING, GB, vol. 37, no. 1, septembre 1998 (1998-09), pages 19-24, XP004141130 ISSN: 0144-8617
- ZUBKOV V A ET AL: "Structure of the capsular polysaccharide from Alteromonas sp. CMM 155" CARBOHYDRATE RESEARCH, ELSEVIER SCIENTIFIC PUBLISHING COMPANY. AMSTERDAM, NL, vol. 275, no. 1, 15 septembre 1995 (1995-09-15), pages 147-154, XP004021934 ISSN: 0008-6215

## Description

La présente invention est relative à l'utilisation de certains dérivés polysaccharidiques hautement sulfatés et de faible masse molaire obtenus à partir d'un polysaccharide d'origine bactérienne pour la préparation d'une composition pharmaceutique destinée à la modulation de l'angiogenèse, utilisable notamment pour accélérer la réparation de l'endothélium vasculaire lors d'accidents thrombotiques et ce avec un risque hémorragique faible.

L'héparine, polysaccharide sulfaté extrait de la muqueuse de mammifères, est l'agent anti-thrombotique le plus utilisé dans la prévention et le traitement de la thrombose veineuse. Cependant, au cours des traitements par l'héparine, il peut survenir des effets secondaires tels qu'une réaction allergique ou une complication hémorragique (due à sa forte activité anticoagulante). De plus, l'héparine s'est révélée peu active dans la prévention et le traitement de la thrombose artérielle. A cause des limites d'utilisation thérapeutique de l'héparine, il est important de pouvoir disposer de nouveaux polysaccharides (par exemple fucanes, chondroïtine sulfate fucosylé, exopolysaccharides, etc...), d'origine non mammalienne (pour éviter tout risque potentiel de transmission d'agents non conventionnels), de faible activité anticoagulante (afin de diminuer le risque hémorragique) et efficaces dans la lutte contre la thrombose artérielle.

Les objectifs sont de prévenir ou de traiter la thrombose artérielle et ses conséquences. En effet, lors d'une lésion vasculaire artérielle, l'absence d'une monocouche de cellules endothéliales déclenche une agrégation plaquettaire suivie de la formation d'un thrombus. Ce thrombus artériel peut obstruer la lumière du vaisseau sanguin et ainsi empêcher toute oxygénation du tissu en aval susceptible d'entraîner une nécrose. Face à ce risque, il serait intéressant de pouvoir favoriser la réparation de l'endothélium vasculaire et de stimuler la formation d'une circulation vasculaire collatérale, deux événements cellulaires provenant de la stimulation de l'angiogenèse.

L'angiogenèse est un processus complexe, physiologiquement régulé, qui aboutit à la formation de nouveaux vaisseaux sanguins à partir du réseau vasculaire pré-existant. C'est lors des situations pathologiques précédemment décrites (lésion de l'endothélium vasculaire ou hypoxie) que la quantité de certains facteurs angiogènes tels que le facteur de croissance fibroblastique basique (FGF-2) ou le facteur de croissance de l'endothélium vasculaire (VEGF) devient bien plus importante dans l'environnement péri-vasculaire. En effet, le FGF-2 est principalement libéré après lésion de l'endothélium vasculaire alors que le VEGF est principalement synthétisé par les cellules en situation d'hypoxie. Ces deux facteurs FGF-2 et VEGF déclenchent le processus d'angiogenèse en agissant sur les cellules endothéliales, ce qui se traduit par une stimulation des trois étapes principales de l'angiogenèse : la migration, la prolifération et la différenciation des cellules endothéliales pour former des néo-vaisseaux (Folkman J., Nat. Med., 1995, 1, 27-31 ; Detillieux K.A. et al., Cardiovasc. Res., 2003, 57, 8-19 ; Heilmann C. et al., Cardiovasc. Surg., 2002, 10, 570-578 ; Mc Neil P.L. et al., J. Cell Biol., 1989, 109, 811-822 ; Heba G. et al., J. Vasc. Res., 2001, 38, 288-300 et Marti H.H. et Risau W. et al., Thromb. Haemost., 1999, 82 (Suppl. 1), 44-52).

Des études antérieures ont montré que des polysaccharides sulfatés (tels que l'héparine ou le fucane) ou non sulfatés (tels que par exemple l'acide hyaluronique) peuvent, selon les conditions expérimentales, moduler l'angiogenèse induite par un facteur de croissance angiogène. Ces polysaccharides sont riches en groupements chimiques de charge négative (groupements sulfate et carboxylique) leur permettant d'interagir avec un grand nombre de protéines de charge positive dont certaines protéines matricielles (membrane basale, matrice extracellulaire), certains facteurs de la coagulation, facteurs de croissance (tels que par exemple le FGF-2 et le VEGF) et leurs récepteurs de haute affinité qui sont des protéines connues pour intervenir dans l'angiogenèse (Giraux J.L. et al., Eur. J. Cell Biol., 1998, 77, 352-359 ; Matou S. et al., Thromb. Res., 2002, 106, 213-221 ; Chabut D. et al., Mol. Pharmacol., 2003, 64, 696-702 ; Luyt C.E. et al., J. Pharmacol. Exp. Ther., 2003, 305, 24-30). Ces facteurs de croissance appartiennent à la famille des facteurs de croissance pouvant se fixer à l'héparine ("Heparin-Binding Growth Factor" ou HBGF). Ces polysaccharides solubles polyanioniques peuvent moduler l'interaction du facteur angiogène avec ses récepteurs de haute affinité (induisant la transduction du signal) ou de basse affinité (cofacteur des récepteurs de haute affinité), présents à la surface des cellules endothéliales. Ainsi, le polysaccharide peut potentialiser l'effet du facteur angiogène en catalysant la fixation de plusieurs molécules aux récepteurs de haute affinité, ce qui amplifie la dimérisation de ces derniers. Cette dimérisation des récepteurs déclenche la transduction du signal stimulant ainsi l'expression des gènes impliqués dans la migration, la prolifération et la différenciation des cellules endothéliales. Dans ce cas de figure, le polysaccharide sulfaté agit comme cofacteur tout comme le protéoglycane à héparane sulfate membranaire (site de basse affinité) (Rusnati M.et Presta M., Int. J. Clin. Lab. Res., 1996, 26, 15-23 ; Soker S. et al., Biochem. Biophys. Res. Commun., 1994, 203, 1339-47). A l'inverse, le polysaccharide sulfaté peut interférer dans la fixation du facteur angiogène au protéoglycane à héparane sulfate par compétition de charge empêchant ainsi l'induction d'une réponse cellulaire.

L'exopolysaccharide (EPS) dénommé GY 785 est un polysaccharide produit par la bactérie *Alteromonas infernus* qui est décrit dans le Brevet FR 2 755 142 ainsi que dans l'article de Raguenes G. et al.,. J. Appl. Microbiol., 1997, 82, 422-30). L'EPS GY 785 est constitué d'une population hétérogène de molécules de masse molaire moyenne élevée, c'est-à-dire de masse molaire supérieure à 1.10⁶ g/mol. L'EPS GY 785 est faiblement sulfaté (taux de sulfate inférieur à 10 % en poids) ; il est constitué d'oses neutres (glucose et galactose majoritairement) et d'oses acides (acide glucuronique et acide galacturonique) ; il ne comprend pas d'osamines et de substituants acétate, lactate, pyruvate et succinates ; sa teneur en protéine est environ de 4 % en poids (Guezennec J., Ind. Microb. Biotech., 2002, 29, 204-208).

L'EPS GY 785 est difficilement utilisable sous sa forme native en thérapeutique, à cause de sa masse molaire élevée et de son hétérogénéité en taille qui entraînent une mauvaise solubilité et qui rendent très difficile la caractérisation des préparations actives et leur obtention reproductible.

Lors de travaux précédents, les Inventeurs ont mis au point un procédé de préparation de dérivés sulfatés et de faible masse molaire, comprenant une première étape de sulfatation de l'EPS GY 785 puis une deuxième étape de dépolymérisation de l'EPS GY 785 sur-sulfaté, par hydrolyse acide ou par dépolymérisation radicalaire pour obtenir des dérivés dépolymérisés présentant un taux de sulfate de 20 ou de 40 % en poids (Guezennec J. et al., Carbohydr. Polym., 1998, 37, 19-24). Contrairement au produit natif, les dérivés obtenus après modification chimique de l'EPS GY 785 présentent des activités anticoagulantes (Colliec-Jouault S. et al., Biochim. Biophys. Acta 1528, 2001, 141-151).

Les Inventeurs ont également montré que la dépolymérisation radicalaire, obtenue par action du peroxyde d'hydrogène en présence d'acétate de cuivre, conduit en une seule étape à des dérivés de faible masse molaire, généralement inférieure à 25 000 g/mol, de composition constante (faible polydispersité de masse molaire) et avec des rendements bien supérieurs à ceux obtenus avec le procédé de dépolymérisation par hydrolyse acide. La réaction procède par la formation de radicaux libres, issus de la réaction d'un ion métallique (Cu²⁺ ou Fe³⁺) avec le peroxyde d'hydrogène (Van Dedem G. et Nielsen J.I., Pharmeuropa, 1990, 3, 202-218). Ces radicaux libres sont très réactifs et susceptibles de dégrader, à pH neutre, les polysaccharides plus efficacement que l'hydrolyse acide.

Les Inventeurs ont en particulier précédemment montré que la sulfatation par voie chimique, réalisée d'après les conditions de T. Nishino et T. Nagumo (Carbohydr. Res., 1992, 229, 355-362), conduit à des dérivés présentant une teneur en sulfate supérieure à celle de l'EPS GY 785 natif (20 à 40 % en poids de sulfate contre 10 % en poids de sulfate, respectivement). L'activité anticoagulante augmente en fonction de la teneur en sulfate et cette activité disparaît si la teneur en sulfate est inférieure à 20 % en poids.

Les Inventeurs ont maintenant découvert que certains dérivés de polysaccharides sulfatés et dépolymérisés de l'EPS GY 785 (obtenus selon un procédé de préparation comportant une première étape de sulfatation de l'EPS GY 785 natif pour obtenir un dérivé sur-sulfaté de l'EPS GY 785 présentant un taux de sulfate inférieur ou égal à 45 % en poids, puis une deuxième étape de dépolymérisation radicalaire du dérivé de l'EPS GY 785 sur-sulfaté obtenu à l'étape précédente, pour conduire à un dérivé de polysaccharide présentant une masse molaire inférieure ou égale à 25 000 g/mol) ont une activité sur la modulation de l'angiogenèse.

La présente Invention a ainsi pour premier objet l'utilisation de dérivés polysaccharidiques sulfatés présentant une masse molaire inférieure ou égale à 25 000 g/mol, un indice de polydispersité inférieur à 2 et un taux de substitution en groupements sulfates inférieur ou égal à 45 %, et de préférence compris entre 30 et 45 % inclusivement, lesdits dérivés étant obtenus selon un procédé de préparation comprenant au moins les étapes suivantes :
- une première étape de sulfatation chimique, comprenant :
   a) une première sous-étape de dissolution, dans un solvant anhydre, d'un polysaccharide lyophilisé GY 785 produit par la bactérie marine *Alteromonas infernus* et
   b) une deuxième sous-étape d'addition, à une température comprise entre 45 et 60°C, d'au moins un agent de sulfatation chimique en une quantité suffisante pour conduire à un polysaccharide sulfaté présentant un taux de substitution en groupements sulfates inférieur ou égal à 45 % en poids par rapport au poids total du polysaccharide GY 785 sulfaté ;
- une deuxième étape de dépolymérisation radicalaire du polysaccharide sulfaté obtenu à l'étape précédente pour conduire à un polysaccharidique sulfaté présentant une masse molaire inférieure ou égale à 25 000 g/mol et un indice de polydispersité inférieur à 2, pour la préparation d'une composition pharmaceutique destinée à une activité pro-angiogène pour la thérapie cardio-vasculaire.

Les dérivés polysaccharidiques sulfatés obtenus selon le procédé utilisé conformément à l'Invention présentent une bonne homogénéité en taille (indice de polydispersité : I(Mp/Mn)<2 avec Mp = masse molaire moyenne en poids et Mn = masse molaire moyenne en nombre), sans qu'il soit nécessaire de procéder à un fractionnement complémentaire par exclusion stérique.

Lors de la première étape, l'EPS GY 785 lyophilisé peut être utilisé à l'état natif ou sous forme de sel d'addition avec une base, faible ou forte, pouvant par exemple être choisie parmi la pyridine, la triéthylamine, la tributylamine, l'hydroxyde de tétrabutylammonium et la soude.

Selon une forme de réalisation préférée du procédé utilisé conformément à l'Invention, l'EPS GY 785 mis en oeuvre lors de la première étape est de préférence sous la forme d'un sel lyophilisé d'addition avec une base. Ce sel lyophilisé peut par exemple être préparé par élution d'une solution aqueuse d'EPS GY 785 à une concentration comprise entre 1 et 8 mg/ml sur une colonne de résine échangeuse d'ions telle que par exemple celles vendues sous la dénomination Dowex ® par la société Dow Chemical ; l'EPS GY 785 est mis sous forme H⁺. L'éluat est collecté tant que le pH reste acide puis le pH est ensuite ajusté à environ 6,5 avec la base désirée telle que définie ci-dessus. L'EPS GY 785 sous forme de sel est alors ultrafiltré et lyophilisé.

Les solvants anhydres pouvant être utilisés lors de la première étape sont de préférence choisis parmi le diméthylformamide (DMF), le diméthylsulfoxyde (DMSO) et le formamide.

La quantité d'EPS GY 785 présente au sein du solvant anhydre peut être comprise entre 1 et 10 mg/ml environ, de préférence entre 1 et 5 mg/ml environ et encore plus préférentiellement cette quantité est de 5 mg/ml environ.

Lors de la sous étape a), la dissolution de l'EPS GY 785 dans le solvant anhydre est de préférence réalisée, sous agitation, à température ambiante pendant 1 à 2 heures environ puis à une température comprise entre 40 et 50 °C, de préférence à une température d'environ 45°C pendant environ 2 heures sous argon avec du tamis moléculaire.

Le ou les agents de sulfatation chimique utilisés lors de la sous étape b) peuvent être additionnés à la solution d'EPS GY 785 sous forme sèche ou sous la forme d'une solution dans le même solvant anhydre que celui utilisé pour dissoudre l'EPS GY 785.

Les agents de sulfatation chimique sont de préférence choisis parmi les complexes de pyridine sulfate (libre ou couplée à un polymère), de triéthylamine sulfate, et de triméthylamine sulfate. Lors de la sous étape b), le ou les agents de sulfatation chimique sont ajoutés à la solution d'EPS GY 785 en une quantité pondérale représentant de préférence de 4 à 6 fois environ et encore plus préférentiellement 5 fois environ la masse de l'EPS GY 785 en solution.

La réaction de sulfatation chimique est alors de préférence conduite sous agitation pendant une durée comprise entre 2 et 24 heures environ selon le degré de sulfatation souhaité.

Lorsque le degré de sulfatation désiré est atteint, la réaction de sulfatation est arrêtée, après refroidissement du milieu réactionnel :
- soit par addition d'eau dans une proportion de préférence égale à 1/10 du volume réactionnel et ajustement du pH du milieu réactionnel à 9 avec un agent alcalinisant tel que par exemple de la soude (3 M) ;
- soit et de préférence, par précipitation en présence d'acétone saturée en chlorure de sodium ou de méthanol puis dissolution du précipité en eau.

Selon une forme de réalisation particulière du procédé conforme à l'Invention, la solution d'EPS GY 785 sulfaté est de préférence ensuite dialysée pour éliminer les différents sels puis lyophilisée.

La deuxième étape de dépolymérisation par voie radicalaire de l'EPS GY 785 sulfaté obtenu à l'issue de la première étape est de préférence réalisée par addition au mélange réactionnel comprenant l'EPS GY 785 sulfaté en présence d'un catalyseur métallique, d'une solution d'un agent oxydant, de préférence choisi parmi les peroxydes tels que le peroxyde d'hydrogène et les peracides tels que l'acide peracétique et l'acide 3-chloro-perbenzoïque ; ladite addition étant effectuée en continu et sous agitation pendant une durée comprise entre 30 minutes et 10 heures ; le mélange réactionnel étant de préférence maintenu à un pH compris entre 6 et 8 par ajout continu d'un agent alcalinisant tel que la soude, et à une température comprise entre 30 et 70°C environ pendant toute la durée de la réaction de dépolymérisation radicalaire.

Lors de cette étape, l'agent oxydant est de préférence une solution de peroxyde d'hydrogène (H₂O₂). Dans ce cas, la solution d'agent oxydant est de préférence ajoutée à un débit compris entre V1/1000 et V1/10 ml/minute, V1 étant le volume du milieu réactionnel contenant le dérivé sulfaté d'EPS GY 785 dans lequel est additionnée la solution d'agent oxydant.

Selon un autre mode de réalisation préféré de la présente Invention, la solution d'agent oxydant utilisée lors de la deuxième étape est une solution de peroxyde d'hydrogène qui présente une concentration comprise entre 0,1 % et 0,5 % en poids environ, de préférence de l'ordre de 0,1 à 0,2 % en poids et qui est ajoutée au mélange réactionnel à un débit compris entre V1/50 et V1/500 ml/minutes, de préférence de l'ordre de V1/100 ml/minutes.

Lors de cette deuxième étape de dépolymérisation, et selon un mode de réalisation préféré de la présente Invention, l'EPS GY 785 sulfaté est présent dans le mélange réactionnel à une concentration comprise entre environ 2 et 10 mg/ml de mélange réactionnel.

Les catalyseurs métalliques utilisables lors de l'étape de dépolymérisation sont de préférence choisis parmi les ions Cu ⁺⁺, Fe ⁺⁺, Cr ⁺⁺⁺ et l'anion Cr₂O₇²⁻ tels que décrits notamment dans la demande de brevet EP-A-0 221 977.

Selon un mode de mise en oeuvre préféré de la présente Invention, le catalyseur métallique est présent dans le mélange réactionnel à une concentration comprise entre 10⁻³ M et 10⁻¹ M environ, et encore plus préférentiellement à une concentration comprise entre 0,001 et 0,05 M environ.

Lorsque la réaction est terminée, le procédé utilisé conformément à l'Invention peut, en outre, comprendre une étape supplémentaire de réduction des dérivés polysaccharidiques obtenus, à l'aide d'un agent réducteur, de façon à stabiliser les chaînes dont les extrémités réductrices sont très réactives et notamment pour éviter une hydrolyse des chaînes par la réaction dite de "peeling".

La nature des agents réducteurs utilisables à cet effet n'est pas critique. Il peut notamment s'agir du borohydrure de sodium.

Lorsque les réactions sont terminées, les dérivés polysaccharidiques résultant de la dépolymérisation peuvent être recueillis par toute technique appropriée bien connue de l'homme du métier telle que par exemple par ultrafiltration sur membrane.

Le procédé de dépolymérisation radicalaire conforme à l'Invention et tel que décrit ci-dessus permet d'obtenir, en une seule étape, sans fractionnement préparatif par chromatographie d'exclusion stérique, et avec un bon rendement, des dérivés polysaccharidiques sulfatés très homogènes, de masse molaire inférieure ou égale à 25 000 g/mol.

Dans le cadre de l'exposé de la présente Invention, on entend par "dérivé homogène", un dérivé qui, en chromatographie d'exclusion stérique haute performance, présente un seul pic principal représentant une population majoritaire de chaînes polysaccharidiques homogènes en taille caractérisée par une Mc = masse chromatographique ou masse au pic proche de 20 000 et un indice de polydispersité (calculé en prenant le rapport Mp/Mn) inférieur à 2 et de préférence compris entre 1,5 et 2.

Ainsi que cela est démontré dans les exemples qui suivent, les dérivés ainsi obtenus présentent une activité de modulation de l'angiogenèse. Ils permettent en particulier de favoriser la réparation de l'endothélium vasculaire avec un faible effet anticoagulant.

La composition pharmaceutiques contenant les dérivés polysaccharidiques obtenus conformément à l'Invention peut en outre être utilisée en association avec un ou plusieurs facteurs de croissance présents au sein de ladite composition pharmaceutique ou présents au sein d'une composition pharmaceutique distincte qui sera alors administrée de façon séparée, c'est-à-dire avant, en même temps ou après l'administration de la composition pharmaceutique renfermant les dérivés polysaccharidiques. De tels facteurs de croissance peuvent en particulier être choisis parmi le FGF, le VEGF, le HGF ("Hepatocyte Growth Factor") et le PGF ("Placenta Growth Factor").

Compte tenu de leurs propriétés de modulation de l'angiogenèse, les dérivés polysaccharidiques tels que définis précédemment peuvent être utilisés pour la préparation d'une composition pharmaceutique à activité pro-angiogène, plus particulièrement destinée à la thérapie cardio-vasculaire, ladite composition permettant notamment de favoriser la revascularisation et le remodelage vasculaire et de prévenir et/ou de traiter l'ischémie.

Les compositions pharmaceutiques obtenues conformément à l'invention sont préférentiellement destinées à être administrées par voie générale (orale, sous-cutanée ou intra-veineuse). Elles peuvent également être administrées localement, sous formes de gels, crèmes, pommades, lotions, etc...

Elles peuvent également être administrées *in situ* par l'intermédiaire de substrats, de dispositifs résorbables ou non, tels que par exemple, des supports à libération différée, ou des éponges à délitement lent.

La présente Invention sera mieux comprise à l'aide du complément de description qui va suivre, et qui se réfère (i) à un exemple de préparation de dérivés polysaccharidiques sulfatés à partir de l'EPS GY 785 (EPS SDR) produit par la bactérie marine d'origine hydrothermale *Alteromonas infernus,* en mettant en oeuvre le procédé tel que défini précédemment, (ii) à un exemple concernant l'effet d'un dérivé EPS SDR sur la prolifération des cellules endothéliales induite par le FGF-2, (iii) à un exemple concernant l'effet d'un dérivé EPS SDR sur la prolifération des cellules endothéliales induite par le VEGF, (iv) à un exemple de mise en évidence de l'effet de l'EPS SDR sur la migration des cellules endothéliales induite par le FGF-2 ou le VEGF, (v) à des exemples étudiant l'effet de l'EPS SDR sur la différenciation des cellules endothéliales induite par le FGF-2 ou le VEGF.

### EXEMPLE 1: PREPARATION D'UN DERIVE DE L'EPS GY 785 HAUTEMENT SULFATE ET DE FAIBLE MASSE MOLAIRE

### 1) Sulfatation chimique de l'EPS GY 785

500 mg de lyophilisat d'EPS GY 785 produit par la bactérie marine d'origine hydrothermale *Alteromonas infernus* selon le procédé décrit à l'exemple 1 du brevet FR 2 755 142, ont été dissous dans 100 ml de DMF anhydre sous agitation douce (250 tours/min) pendant 2 heures à température ambiante, puis pendant 2 heures à une température de 45 °C.

Lorsque la dissolution est complète, 2,5 g de complexe de pyridine-SO₃ vendu sous la référence 84737 par la société Fluka (soit 5 fois la masse du polysaccharide GY 785) ont été ajoutés au milieu réactionnel. La température du mélange a ensuite été portée et maintenue à 45 °C pendant 2 heures sous agitation. Le mélange réactionnel a été transféré dans un bécher. La réaction a alors été stoppée par addition de 40 ml d'eau, puis le pH a été amené à 9 avec de la soude 3 M. Le mélange réactionnel a alors été dialysé sur un boudin de dialyse présentant un seuil de coupure compris entre 12000 et 16 000 Da contre de l'eau du robinet (1 nuit avec de l'eau courante) puis 3 fois pendant 24 heures contre de l'eau Milli-Q.

Après dialyse, la solution contenant l'EPS GY 785 sulfaté a été congelée et lyophilisée.

### 2) Dépolymérisation radicalaire et réduction au borohydrure de sodium

400 mg d'EPS GY 785 sulfaté obtenu ci-dessus à l'étape précédente ont été dissous dans 95 ml d'eau. Après dissolution, on a ajouté 2 ml d'une solution catalytique contenant 36 mg d'acétate de cuivre monohydraté (10⁻³ M). La température du réacteur est alors portée à 60°C et le pH est ajusté à 7,5 par addition de soude 1 M. Une solution à 0,115 % (v/v) de peroxyde d'hydrogène a alors été ajoutée à un débit de 1 ml par minute, et le pH a été régulé autour de 7,5 par addition de soude 1 M. La réaction a été arrêtée au bout de 1 heure.

La réduction est réalisée en fin de dépolymérisation par addition dans le réacteur de borohydrure de sodium (270 mg de Na BH₄ dissous dans 10 ml d'eau). La réduction se déroule sous agitation pendant 2 heures à température ambiante. La réduction est stoppée par addition d'acide acétique 10 N qui permet d'éliminer l'excès de Na BH₄ restant sous forme de dégagement gazeux d'hydrogène. La solution a ensuite été filtrée sur Büchner avec des filtres en microfibres de verre (porosité 3 µm). La solution filtrée a été éluée sur une colonne CHELEX® 20 (BIORAD) afin d'éliminer le cuivre résiduel. La solution décontaminée est ensuite ultrafiltrée sur cassette (seuil de coupure 1 000 Da) puis lyophilisée.

### 3) Caractérisation du dérivé sulfaté puis dépolymérisé de l'EPS GY 785 (EPS SDR) ; comparaison avec l'EPS GY 785 natif

Les masses molaires (Mc : Masse molaire chromatographique déterminée au sommet du pic; Mp et Mn) et la polydispersité (I = Mp/Mn) du dérivé sulfaté puis dépolymérisé de l'EPS GY 785 obtenu ci-dessus ont été déterminées par chromatographie d'exclusion stérique haute performance (HPSEC) sur une chaîne Kontron, dans l'acétate d'ammonium aqueux 0,1 M à un débit de 0,5 ml/min en utilisant une colonne Superdex® 200 (PHARMACIA). La colonne a été calibrée avec des étalons polysaccharidiques suivants : pullulanes : 853 000-5 800 g/mol (Polymer Laboratories, Interchim), dextrane : 1 500 g/mol ; mélézitose : 522 g/mol (FLUKA), sucrose : 342 g/mol ; glucose: 180 g/mol (SIGMA). Les résultats sont analysés en utilisant le logiciel Aramis® (JMBS Développement, Le Fontanil, France).

La teneur en oses neutres a été déterminée par la méthode de Tillmans et Philippi (Analyt. Chem., 1929, 28, 350-) modifiée par Rimington (Biochem. J., 1931, 25, 1062-1071).

La teneur en acides uroniques a été établie en utilisant une modification de la méthode m-hydroxydiphényle-H₂SO₄ (Filisetti-Cozzi et Carpitta, Anal. Biochem., 1991, 197, 157-162) et en utilisant l'acide glucuronique comme étalon. L'interférence d'hexoses neutres a été évitée en utilisant du sulfamate de potassium et en procédant à des contrôles comprenant tous les réactifs à l'exception du m-hydroxydiphényle.

Les teneurs en oses neutres et acides ont été déterminées par chromatographie en phase gazeuse. L'analyse des résidus glycosidiques sous forme de dérivés triméthylsilylés a été réalisée selon la méthode de Kamerling et al. (Biochem. J., 1975, 151, 491-495) et modifiée par Montreuil et al. (Glycoproteins In : Carbohydrate analysis, a practical approach, 1986, Chaplin M.F. and Kennedy J.F. (eds), IRL Press, Oxford, 143-204).

Les contenus en sulfates totaux (libres plus liés au dérivé sulfaté puis dépolymérisé de l'EPS GY 785 et de l'EPS GY 785 natif) ont été déterminés par analyse élémentaire du soufre (S %), et en appliquant la relation suivante : pourcentage de groupes sulfate (%) = 3,22 x S %. Le taux de sulfates libres est quantifié par chromatographie échangeuse d'ions sur un système Dionex® DX-500 relié à un conductimètre et suivant la méthode décrite par le fabricant Dionex. Le résultat obtenu permet de calculer le taux de sulfates réellement liés au dérivé d'EPS qui est égal au taux de sulfate totaux (obtenu par analyse élémentaire) moins le taux de sulfates libres (obtenu par chromatographie échangeuse d'ions).

La spectroscopie Infrarouge à transformée de Fourier (FT-IR) a été réalisée sur un Vector 22 possédant une résolution de 4 cm⁻¹, Les spectres infrarouges des polysaccharides ont été réalisés en pastilles de KBr (2 mg de polysaccharide sont mélangés à 200 mg de KBr sec), tous les spectres infrarouges ont été enregistrés entre 4000 et 400 cm⁻¹.

Les activités anticoagulantes du dérivé sulfaté puis dépolymérisé de l'EPS GY 785 et de l'EPS GY 785 natif ont été déterminées par mesure du Temps de Céphaline Activée (TCA) en utilisant le kit TCA (Organon Teknika). Pour ce faire, 100 µl d'un tampon de contrôle, ou de dilutions du dérivé sulfaté puis dépolymérisé de l'EPS GY 785 ou de l'EPS GY 785 natif (0 à 100 µg/ml), sont mélangés avec 100 µl de plasma pauvre en plaquettes (PPP) et 100 µl de réactif TCA. L'ensemble est incubé pendant 3 minutes à 37°C. Le temps de formation du caillot est mesuré après l'addition de 100 µl d'une solution de CaCl₂ à 25 mM.

### 4) Résultats

Les résultats obtenus ont été rassemblés dans le tableau I ci-après:

**TABLEAU I**

| **Caractéristiques** | **Dérivé sulfaté et dépolymérisé de l'EPS GY 785 (EPS SDR)** | **EPS GY 785 natif** |
|---|---|---|
| Rendement (%) | 70 | - |
| Oses totaux (g/100 g)¹ | 31 | 58 |
| Oses acides (g/100 g)¹ | 20 | 29 |
| Rhamnose ² | 1,7 | 2,7 |
| Mannose ² | 1,6 | 2,8 |
| Fucose ² | 0,5 | 1,3 |
| Xylose | 0,2 | 0,3 |
| Galactose ² | 7,3 | 12,7 |
| Glucose² | 9,4 | 17 |
| Acide Galacturonique ² | 1,5 | 3,2 |
| Acide Glucuronique ² | 2,3 | 6 |
| -SO₃Na total (g/100 g) ³ | 40 | 10 |
| SO₃Na lié (g/100 g) ⁴ | 37 | - |
| Mc (g/mol) | 22430 | >10⁶ |
| Mp (G/mol) | 20240 | - |
| Mn (g/mol) | 11240 | - |
| I (Mp/Mn) | 1,8 | - |
| Activité anticoagulante⁵ | 10-20 | inactif |

| | | |
|---|---|---|
| ¹ : Dosages colorimétriques ² : Dosage par chromatographie en phase gazeuse ³ : Dosage par analyse élémentaire ⁴ : Dosage par chromatographie échangeuse d'ions ⁵ : Quantité de polysaccharide en µg/ml de plasma humain nécessaire pour doubler le temps de coagulation contrôle, TCA (temps contrôle = 40 secondes). | | |

Ces résultats montrent que le dérivé sulfaté puis dépolymérisé de l'EPS GY 785 est obtenu avec un bon rendement et que les teneurs en oses sont proportionnellement équivalentes entre le dérivé sulfaté et l'EPS natif naturellement peu sulfaté. La teneur en soufre du produit sur-sulfaté est 4 fois supérieure à celle du produit natif. Par dépolymérisation radicalaire, on obtient en 1 heure un dérivé de masse molaire (Mc et Mp) égale à 20 000 g/mol, homogène en taille (I<2) à partir de l'EPS GY 785 sur-sulfaté de très haute masse molaire (>10⁶ g/mol). Seul le dérivé sulfaté puis dépolymérisé de l'EPS GY 785 est anticoagulant ; il double le temps de coagulation contrôle pour une concentration proche de 15 µg/ml. A titre comparatif, dans les mêmes conditions il faut 4 µg/ml d'héparine de bas poids moléculaire et 1,5 µg/ml d'héparine non fractionnée pour obtenir ce doublement. Ainsi le dérivé sulfaté puis dépolymérisé de l'EPS GY 785 est respectivement 4 et 10 fois moins anticoagulant qu'une héparine de bas moléculaire ou qu'une héparine non fractionnée.

### EXEMPLE 2 : EFFET DU DERIVE DE L'EPS GY 785 HAUTEMENT SULFATE ET DE FAIBLE MASSE MOLAIRE SUR LA PROLIFERATION DES CELLULES ENDOTHELIALES INDUITE PAR LE FGF-2

L'effet du dérivé sulfaté et de faible masse molaire de l'EPS GY 785 (EPS SDR) conforme à l'Invention et tel que préparé ci-dessus à l'exemple 1 sur la prolifération des cellules endothéliales extraites de la veine de cordon ombilical humain (HUVEC) a été comparé à celui du dérivé natif dépolymérisé (EPS DR), c'est-à-dire ayant été obtenu par dépolymérisation radicalaire.

### a) Préparation de l'EPS DR

Ces dérivés ont été préparés à partir de lyophilisat d'EPS GY 785 produit par la bactérie marine *Alteromonas infernus* selon le procédé décrit à l'exemple 1 du brevet FR 2 755 142.

L'EPS DR a été préparé selon un procédé ne mettant en oeuvre que l'étape de dépolymérisation radicalaire telle que décrite ci-dessus au paragraphe 2) de l'exemple 1.

Les caractéristiques des dérivés EPS DR ont été déterminées selon les méthodes décrites ci-dessus à l'exemple 1 et sont résumées dans le Tableau II ci-après :

**TABLEAU II**

| **Caractéristiques** | **EPS DR** |
|---|---|
| Oses totaux (g/100 g)¹ | 51 |
| Oses acides (g/100 g)¹ | 38 |
| -SO₃Na total (g/100 g)² | 10 |
| Mc (g/mol) | 7800 |
| Mp (g/mol) | 17300 |
| Mn (g/mol) | 6000 |
| I (Mp/Mn) | 2,8 |
| Activité anticoagulante ³ | inactif |

| | |
|---|---|
| ¹ : Dosages colorimétriques ² : Dosage par analyse élémentaire ³ : Quantité de polysaccharide en µg/ml de plasma humain nécessaire pour doubler le temps de coagulation contrôle, TCA (temps contrôle = 40 secondes) ; nd : non déterminé | |

Le dérivé EPS DR est moins sulfaté que le dérivé SDR. L'analyse HPSEC indique que le dérivé EPS DR est moins homogène en taille que le dérivé EPS SDR avec une polydispersité de 2,8 contre 1,8. Les chaînes polysaccharidiques composant le dérivé EPS DR sont majoritairement plus petites (Mc = 7800) que celles du dérivé EPS SDR (Mc = 22430). Le dérivé DR n'a aucune activité coagulante, alors que l'activité du dérivé EPS SDR a une activité coagulante pour doubler le temps de coagulation contrôle, TCA (temps contrôle = 40 secondes), de 10-20 µg de produit/ml de plasma.

### b) Protocole

Les cellules HUVEC sont ensemencées dans des puits recouverts de gélatine à 0,5 %. Après 24 heures d'incubation, le milieu de culture est changé : milieu contrôle seul (milieux de culture M199 (1 volume) + RPMI 1640 (1 volume) vendus par la société Gibco-BRL, Cergy-Pontoise, France, supplémenté par 5 % de sérum de veau foetal vendu par la société ATGC, Noisy-le-Grand, France) ou enrichi en FGF-2 à 5 ng/ml (vendu par la société AbCys SA, Paris, France) ou en dérivés d'EPS seuls (10 µg/ml) ou en FGF-2 à 5 ng/ml) + dérivés d'EPS (1 ou 10 µg/ml). Le milieu est renouvelé tous les 2 jours. Après 3 jours de traitement, les cellules sont détachées et comptées à l'aide d'une cellule de Malassez.

### c) Résultats

Les résultats sont présentés dans le Tableau III ci-après :

**TABLEAU III**

| **Facteurs de croissance et/ou polysaccharides** | **Augmentation de la prolifération cellulaire (en % par rapport au FGF-2)** |
|---|---|
| Contrôle (sans FGF-2) | 66,8 ± 5,3 **** |
| FGF-2 | 100 |
| FGF-2 + EPS SDR (1 µg/ml) | 107,3 ± 1,4 |
| FGF-2 + EPS SDR (10 µg/ml) | 120,9 ± 5,2 * |
| FGF-2 + EPS DR (1 µg/ml) ⁶ | 89,6 ± 2,9 |
| FGF-2 + EPS DR (10 µg/ml) ⁶ | 112,8 ± 6,9 |

| | |
|---|---|
| ⁶ :polysaccharides ne faisant pas partie de l'Invention * : p< 0,05 et ****: p< 0,0001 | |

Dans le Tableau III ci-dessus, chaque valeur représente la moyenne ± l'écart type de cinq déterminations. Pour chaque expérience, les résultats sont exprimés en %, 100 % correspondant aux cellules traitées par FGF- 2 seul à 5 ng /ml.

Ces résultats montrent que, comparativement au contrôle (absence de facteur de croissance et de polysaccharide) et à l'ajout de FGF-2 seul, on observe une augmentation significative de la prolifération cellulaire en présence de FGF-2 et d'EPS SDR à la concentration de 10 µg/ml.

### EXEMPLE 3 : EFFET DU DERIVE DE L'EPS GY 785 SDR SUR LA PROLIFERATION DES CELLULES ENDOTHELIALES INDUITE PAR LE VEGF

L'effet du dérivé de l'EPS GY 785 tel que synthétisé ci-dessus à l'exemple 1 sur la prolifération des cellules endothéliales induite par le VÉGF (vendu par la société AbCys SA, Paris, France) a également été étudié selon le même protocole que celui utilisé ci-dessus à l'exemple 2.

Dans cet exemple, le VEGF a été utilisé à une concentration de 10 ng/ml et les cellules HUVEC ont été détachées et comptées après 6 jours de traitement.

Les résultats obtenus sont présentés dans le Tableau IV ci-après :

**TABLEAU IV**

| Facteurs de croissance ou polysaccharide | Augmentation de la prolifération cellulaire (en % par rapport au VEGF) |
|---|---|
| Contrôle (sans VEGF) | 59,5 ± 4,9 **** |
| VEGF | 100 |
| VEGF + EPS SDR (10 µg/ml) | 137,6 ± 10,7 ** |

| | |
|---|---|
| ** : p<0,01 et **** : p<0,0001. | |

Dans le Tableau IV ci-dessus, chaque valeur représente la moyenne ± l'écart type de 4 déterminations. Pour chaque expérience, les résultats sont exprimés en %, 100 % correspondant aux cellules traitées par le VEGF seul à 10 ng/ml.

### EXEMPLE 4 : MISE EN EVIDENCE DE L'EFFET DE L'EPS SDR SUR LA MIGRATION DES CELLULES ENDOTHELIALES INDUITE PAR LE FGF-2 OU LE VEGF

### 1) Protocole

Dans cet exemple le dérivé EPS SDR tel que préparé ci-dessus à l'exemple 1 a été utilisé.

Les facteurs de croissance angiogènes tels que le FGF-2 et le VEGF exercent un effet chimio-attracteur sur les cellules endothéliales, ce qui déclenche leur migration. Un système qui met en évidence la migration orientée des cellules endothéliales induite par le facteur angiogène a été utilisé par les Inventeurs. Il s'agit du système de la chambre de Boyden. Ce système comprend deux compartiments : une chambre supérieure ou insert contenant un milieu de culture (milieu de culture identique à celui qui a été décrit en b) de l'exemple 2 ci-dessus) pauvre en facteurs angiogènes (FGF-2 : 1 ng/ml ; et VEGF : 1 ng/ml) et une chambre inférieure ou puits contenant un milieu de culture identique à celui de la chambre supérieure mais riche en facteurs angiogènes (FGF-2 : 10 ng/ml ; et VEGF : 10 ng/ml), ces deux chambres étant séparées par une membrane poreuse. Des cellules endothéliales sont déposées sur la face supérieure de membrane poreuse séparant les deux chambres. Sous l'effet du gradient de concentrations en facteur angiogène ainsi créé entre les deux compartiments, les cellules endothéliales sont attirées vers le compartiment le plus concentré en facteur angiogène (chambre inférieure).

Ajoutés ou non dans les deux compartiments de la chambre de Boyden, l'effet du dérivé EPS SDR à 10 µg/ml, sur la migration orientée des cellules endothéliales, a été évalué.

Grâce à cette migration orientée et après 6 heures d'incubation, une partie des cellules est localisée sur la face inférieure de la membrane. Chaque condition a été testée 3 fois en "duplicata" à chaque fois. Seules les cellules qui ont migré (localisées sur la face inférieure) sont fixées et colorées en vue d'un comptage au microscope optique.

### 2) Résultats

En présence de facteurs de croissance seuls (FGF-2 ou VEGF), c'est-à-dire sans dérivé d'EPS SDR, la migration des cellules est augmentée significativement de 40 % (p<0,0001) par rapport au contrôle (migration des cellules à partir d'un milieu de culture ne contenant ni facteur de croissance ni dérivé d'EPS). L'addition de 10 µg/ml du dérivé EPS SDR au FGF-2 diminue significativement de 20 % (p<0,05) la migration des cellules par rapport à celle induite par le FGF-2 seul. L'addition du même dérivé à la même concentration au VEGF ne modifie pas la migration des cellules par rapport à celle observée en présence de VEGF seul.

### EXEMPLE 5 : EFFET DE L'EPS SDR SUR LA DIFFERENCIATION DES CELLULES ENDOTHELIALES INDUITE PAR LE FGF-2 - COMPARAISON AVEC L'EPS DR

Dans cet exemple, on a utilisé l'EPS SDR tel que préparé ci-dessus à l'exemple 1, ainsi que l'EPS DR tel que préparé ci-dessus à l'exemple 2.

### 1) Protocoles

### a) Angiogenèse in vitro sur Matrigel^{®}

A partir d'un modèle d'angiogenèse *in vitro,* il a été montré que le fucane (polysaccharide sulfaté d'algue brune), contrairement à l'héparine non fractionnée, potentialise l'effet angiogène du FGF-2 sur des cellules endothéliales humaines de macro-vaisseaux (Matou *et al.,* 2002, précité).

Le même système expérimental que celui utilisé dans l'article de Matou *et al.* a été utilisé dans cet exemple pour l'étude de l'effet des EPS vis-à-vis du FGF-2.

Ce système consiste, dans un premier temps, à traiter des cellules endothéliales HUVEC avec le FGF-2, en présence du dérivé d'EPS (EPS SDR, EPS DR) pendant 72 heures. Pour cela, les cellules sont ensemencées dans des puits recouverts de gélatine à 0,5 %. La concentration de FGF-2 utilisée est de 5 ng/ml, concentration la plus utilisée dans les modèles *in vitro* d'angiogenèse et celle des EPS est de 10 µg/ml.

Puis, les cellules ainsi traitées sont détachées par du Versène® (Gibco) et de la collagénase à 0,01 %, et déposées (en l'absence de polysaccharides et/ou du facteur angiogène) sur une membrane basale reconstituée appelée Matrigel® (vendue par la société Becton Dickinson, Bedford, MA, USA) ; ce système permet d'évaluer l'effet de l'EPS testé sur la différenciation des cellules endothéliales induite par le FGF-2.

Au contact de cette membrane basale, les cellules endothéliales vont (ou non) s'organiser en structure tubulaire, selon leur degré de différenciation. Après 18 heures d'incubation à une température de 37°C, les cellules sont fixées à la glutaraldéhyde puis colorées au Giemsa.

La longueur des tubes vasculaires formés est quantifiée par analyse d'image grâce à un logiciel de mesure (Mesurim® fourni par l'Université d'Amiens, http://www.ac-amiens.fr). Pour chaque puits, une numérisation de 5 champs (4 quadrants et le centre) est effectuée.

### b) Sur-expression des sous-unités d'intégrine α6

Il a été montré antérieurement que l'effet d'un polysaccharide sulfaté (famille des fucanes) sur la différenciation des HUVEC s'accompagnait de la sur-expression de la sous-unité d'intégrine α6 (Matou *et al*, 2002, précité). La sur-expression de la sous-unité d'intégrine α6 a été quantifiée à la surface des cellules endothéliales HUVEC cultivées sur gélatine en l'absence ou en présence de FGF-2 et avec ou sans dérivé EPS SDR, et détachées comme décrit ci-dessus. La quantification a été faite par cytométrie de flux. Les cellules ont été incubées pendant 30 minutes à une température de 4°C avec l'anticorps anti-α6 (vendu par la société BD Biosciences & Pharmingen, San Diego, CA, USA sous la référence 555736). Puis les cellules ont été analysées par immunofluorescence sur un cytomètre de flux FACSCalibur® (BD Biosciences).

### 2) Résultats

Les résultats obtenus sont reportés dans le Tableau V ci-après :

**TABLEAU V**

| **Facteurs de croissance et/ou polysaccharides** | **Différenciation cellulaire induite par le FGF-2 (en % par rapport au FGF-2)** |
|---|---|
| Contrôle (sans FGF-2) | Aucun tube formé |
| FGF-2 | 100 |
| FGF-2 + EPS SDR (10 µg/ml) | 156,2 ± 4,1** |
| FGF-2 + EPS DR (10 µg/ml) ⁶ | 109,6 ± 13,2 |

| | |
|---|---|
| ⁶ : Dérivé ne faisant pas partie de l'Invention ** : p<0,01. | |

Chaque valeur représente la moyenne ± l'écart type de 3 déterminations.

Ces résultats montrent qu'en l'absence de facteur de croissance et de dérivés d'EPS (contrôle), les cellules ne forment pas de tubes vasculaires après 18 heures sur Matrigel®. En présence de FGF-2 seul, les cellules s'organisent en tubes vasculaires pour former un réseau tridimensionnel vasculaire de type capillaire. Le FGF-2 et le dérivé EPS SDR ajoutés conjointement à la culture cellulaire permettent d'augmenter significativement la densité du réseau. En effet par addition d'un dérivé polysaccharidique obtenu conformément au procédé tel qu'utilisé selon la présente invention aux cellules traitées au FGF-2, la longueur totale des structures tubulaires est significativement augmentée de 56 % (p<0,01) par rapport à celle obtenue en présence du FGF-2 seul.

A titre comparatif, aucune augmentation significative de la formation des tubes vasculaires induite par le FGF-2 n'a été obtenue avec les dérivés EPS DR obtenus selon des procédés ne faisant pas partie de l'Invention.

En ce qui concerne l'expression des sous-unités d'intégrine α6, seul le dérivé d'EPS SDR obtenu selon le procédé mis en oeuvre selon l'Invention a été testé. Le traitement des cellules avec le FGF-2 seul induit une sur-expression significative (p<0,0001) des sous-unités d'intégrine α6 ; cette expression est 4 fois supérieure à celle du témoin (cellules non traitées). Ajouté au FGF-2, le dérivé EPS SDR conforme à l'Invention augmente significativement de 72 % (p<0,001) l'expression de la sous-unité d'intégrine α6 par rapport à celle observée en présence de FGF-2 seul. Le dérivé EPS SDR n'a aucun effet en l'absence de FGF-2.

L'ensemble de ces résultats montre que seul le dérivé d'EPS SDR obtenu selon le procédé de préparation utilisé conformément à l'Invention, c'est à dire dans lequel l'étape de sulfatation chimique précède l'étape de dépolymérisation radicalaire, potentialise l'effet angiogène du FGF-2 de façon significative et reproductible.

### EXEMPLE 6 : EFFET DE L'EPS SDR SUR LA DIFFERENCIATION DES CELLULES ENDOTHELIALES INDUITE PAR LE VEGF

### 1) Protocoles

### a) Angiogenèse in vitro sur Matrigel^{®}

Le VEGF, autre facteur angiogène également très important, est connu pour être plus spécifique des cellules endothéliales que le FGF-2. Le système d'étude mis en place pour évaluer l'effet de FGF-2 sur la différenciation des cellules endothéliales sur Matrigel® et tel que décrit ci-dessus à l'exemple 5 ne permet pas d'observer une formation de tubes vasculaires en présence de VEGF.

Le changement de support protéique a donc été envisagé et du collagène de type I (extrait de la queue de rat, vendu par la société Biogenesis, Poole, UK) a été choisi pour remplacer la gélatine du système décrit dans l'exemple 5. Dès le lendemain de l'ensemencement des cellules HUVEC, le milieu de culture est changé par du milieu enrichi ou non en VEGF (10 ng/ml) avec ou sans l'EPS SDR à raison de 10 µg/ml).

Après 6 jours de ce traitement, les cellules sont détachées par du Versène® (Gibco) et de la collagénase à 0,1 % puis déposées sur Matrigel^{®}. Les structures tubulaires sont observées comme décrit dans l'exemple 5 ci-dessus.

### b) Sur-expression des sous-unités d'intégrine α6

Une quantification de la sous-unité d'intégrine α6 a été effectuée par cytométrie en flux comme décrit ci-dessus dans l'exemple 5.

### 2) Résultats

Sur Matrigel^{®}, les cellules non traitées au VEGF en présence ou non de dérivé d'EPS SDR ne forment pas de structure tubulaire. En présence de VEGF seul, les cellules forment un réseau tridimensionnel vasculaire partiel. L'ajout du dérivé EPS SDR au VEGF augmente la densité de ce réseau.

Le traitement des cellules par le VEGF augmente significativement l'expression de la sous-unité d'intégrine α6 d'un facteur 2 (p<0.001), comparativement aux cellules non traitées. L'ajout du dérivé EPS SDR à ce traitement au VEGF, augmente significativement de 54 % (p<0,001) l'expression de la sous-unité d'intégrine α6 par rapport à celle observée en présence de VEGF seul.

L'ensemble de ces résultats montre que le dérivé EPS SDR obtenu selon un procédé de préparation dans lequel l'étape de sulfatation précède l'étape de dépolymérisation peut être utilisé pour la préparation d'un médicament destiné à moduler l'angiogenèse.

## Revendications

1. Utilisation de dérivés polysaccharidiques sulfatés présentant une masse molaire inférieure ou égale à 25 000 g/mol, un indice de polydispersité inférieur à 2 et un taux de substitution en groupements sulfate inférieur ou égal à 45%, lesdits dérivés étant obtenus selon un procédé de préparation comprenant au moins les étapes suivantes :
- une première étape de sulfatation chimique, comprenant :
a) une première sous étape de dissolution, dans un solvant anhydre, d'un polysaccharide lyophilisé GY 785 produit par la bactérie marine *Alteromonas infernus* et
b) une deuxième sous étape d'addition, à une température comprise entre 45 et 60°C, d'au moins un agent de sulfatation chimique en une quantité suffisante pour conduire à des dérivés polysaccharidiques sulfatés présentant un taux de substitution en groupements sulfates inférieur ou égal 45% en poids par rapport au poids total du polysaccharide GY 785 sulfaté ;
- une deuxième étape de dépolymérisation radicalaire du polysaccharide sulfaté obtenu à l'étape précédente pour conduire à un polysaccharide sulfaté présentant une masse molaire inférieure ou égale à 25 000 g/mol et un indice de polydispersité inférieur à 2,
pour la préparation d'une composition pharmaceutique destinée à une activité pro-angiogène pour la thérapie cardio-vasculaire.

2. Utilisation selon la revendication 1, **caractérisée par le fait que** lors de la première étape, l'EPS GY 785 lyophilisé est utilisé à l'état natif ou sous forme de sel d'addition avec une base.

3. Utilisation selon la revendication 2, **caractérisée par le fait que** l'EPS GY 785 lyophilisé est utilisé sous la forme d'un sel d'addition avec une base forte ou faible choisie parmi la pyridine, la triéthylamine, la tributylamine, l'hydroxyde de tétrabutylammonium et la soude.

4. Utilisation selon l'une quelconque des revendications précédentes, **caractérisée par le fait que** le solvant anhydre est choisi parmi le diméthylformamide, le diméthylsulfoxide et le formamide.

5. Utilisation selon l'une quelconque des revendications précédentes, **caractérisée par le fait que** la quantité d'EPS GY 785 présente au sein du solvant anhydre est comprise entre 1 et 10 mg/ml/

6. Utilisation selon la revendication 5, **caractérisée par le fait que** la quantité d'EPS GY 785 présente au sein du solvant anhydre est comprise entre 1 et 5 mg/ml.

7. Utilisation selon l'une quelconque des revendications précédentes, **caractérisée par le fait que** lors de la sous étape a), la dissolution de l'EPS GY 785 dans le solvant anhydre est réalisée, sous agitation, à température ambiante pendant 1 à 2 heures puis à une température comprise entre 40 et 50°C, pendant 2 heures sous argon avec du tamis moléculaire.

8. Utilisation selon l'une quelconque des revendications précédentes, **caractérisée par le fait que** le ou les agents de sulfatation chimique utilisés lors de la sous étape b) sont choisis parmi les complexes de pyridine sulfate, de triéthylamine sulfate, et de triméthylamine sulfate.

9. Utilisation selon l'une quelconque des revendications précédentes, **caractérisée par le fait que** lors de la sous étape b), le ou les agents de sulfatation chimique sont ajoutés à la solution d'EPS GY 785 en une quantité pondérale représentant de 4 à 6 fois la masse de l'EPS GY 785 en solution.

10. Utilisation selon l'une quelconque des revendications précédentes, **caractérisée par le fait que** la réaction de sulfatation chimique est conduite sous agitation pendant une durée comprise entre 2 et 24 heures.

11. Utilisation selon l'une quelconque des revendications précédentes, **caractérisée par le fait que** la réaction de sulfatation chimique est arrêtée, après refroidissement du milieu réactionnel :
- soit par addition d'eau dans une proportion égale à 1/10 du volume réactionnel et ajustement du pH du milieu réactionnel à 9 avec un agent alcalinisant ;
- soit par précipitation en présence d'acétone saturée en chlorure de sodium ou de méthanol puis dissolution du précipité en eau.

12. Utilisation selon l'une quelconque des revendications précédentes, **caractérisée par le fait que** la deuxième étape de dépolymérisation radicalaire est réalisée par addition au mélange réactionnel comprenant l'EPS GY 785 sulfaté en présence d'un catalyseur métallique, d'une solution d'un agent oxydant ; ladite addition étant effectuée en continu et sous agitation pendant une durée comprise entre 30 minutes à 10 heures.

13. Utilisation selon la revendication 12, **caractérisée par le fait que** le mélange réactionnel est maintenu à un pH compris entre 6 et 8 par ajout continu d'un agent alcalinisant, et à une température comprise entre 30 et 70°C pendant toute la durée de la réaction de dépolymérization radicalaire.

14. Utilisation selon la revendication 12 ou la revendication 13, **caractérisée par le fait que** l'agent oxydant est choisi parmi les peroxydes et les peracides.

15. Utilisation selon la revendication 14, **caractérisée par le fait que** l'agent oxydant est une solution de peroxyde d'hydrogène qui est ajoutée à un débit compris entre V1/1000 et V1/10 ml/minute, V1 étant le volume du milieu réactionnel contenant le dérivé sulfaté d'EPS GY 785 dans lequel est additionné la solution de peroxyde d'hydrogène.

16. Utilisation selon la revendication 14, **caractérisée par le fait que** la solution d'agent oxydant utilisée lors de la deuxième étape est une solution de peroxyde d'hydrogène qui présente une concentration comprise entre 0, 1 % et 0,5% en poids et qui est ajoutée au mélange réactionnel à un débit compris entre V1/50 et V1/500 ml/minutes.

17. Utilisation selon l'une quelconque des revendications précédentes, **caractérisée par le fait que** lors de la deuxième étape de dépolymérisation, l'EPS GY 785 sulfaté est présent dans le mélange réactionnel à une concentration comprise entre 2 et 10 mg/ml de mélange réactionnel.

18. Utilisation selon l'une quelconque des revendications 12 à 17, **caractérisée par le fait que** les catalyseurs métalliques utilisables lors de l'étape de dépolymérisation sont de préférence choisis parmi les ions Cu⁺⁺, Fe⁺⁺, Cr⁺⁺⁺ et l'anion Cr₂O₇²⁻.

19. Utilisation selon l'une quelconque des revendications 12 à 18, **caractérisée par le fait que** le catalyseur métallique est présent dans le mélange réactionnel à une concentration comprise entre 10⁻³ M et 10⁻¹ M.

20. Utilisation selon l'une quelconque des revendications précédentes, **caractérisée par le fait que** le procédé de préparation comprend une étape supplémentaire de réduction des dérivés polysaccharidiques obtenus à l'aide d'un agent réducteur.

21. Utilisation selon l'une quelconque des revendications précédentes, **caractérisée par le fait que** la composition pharmaceutique est utilisée en association avec un ou plusieurs facteurs de croissance.

22. Utilisation selon l'une quelconque des revendications 1 à 21, **caractérisée par le fait que** ladite composition pharmaceutique est destinée à prévenir et/ou à traiter l'ischémie.

23. Utilisation selon l'une quelconque des revendications 1 à 21, **caractérisée par le fait que** ladite composition pharmaceutique est destinée à favoriser la revascularisation et le remodelage vasculaire.

## Claims

1. Use of sulfated polysaccharide derivatives with a molecular weight of less than or equal to 25,000 g/ml, a polydispersity index of less than 2 and a degree of sulfate-group substitution of less than or equal to 45%, said derivatives being obtained according to a preparation process comprising at least the following steps:
- a first step of chemical sulfation, comprising :
a) a first substep consisting of dissolution, in an anhydrous solvent, of a lyophilized GY 785 polysaccharide produced by the marine bacterium *Alteromonas infernus,* and
b) a second substep of addition, at a temperature of between 45 and 60°C, of at least one chemical sulfation agent in an amount sufficient to yield sulfated polysaccharide derivatives having a degree of sulfate-group substitution of less than or equal to 45% by weight relative to the total weight of the sulfated polysaccharide;
- a second step of radical depolymerisation of the sulfated polysaccharide obtained in the previous step, so as to yield a sulfated polysaccharide having a a molecular weight of less than or equal to 25,000 g/ml and a polydispersity index of less than 2,
for the preparation of a pharmaceutical composition with a pro-angiogenic activity for cardiovascular therapy.

2. Use according to claim 1, **characterized in that** in the first step, the lyophilized EPS GY 785 polysaccharide is used in the native state or in the form of an addition salt with a base.

3. Use according to claim 2, **characterized in that** the lyophilized EPS GY 785 polysaccharide is used in the form of an addition salt with a strong or weak base chosen from pyridine, triethylamine, tributylamine, tetrabutylammonium hydroxide and sodium hydroxide.

4. Use according to any one of the previous claims, **characterized in that** the anhydrous solvent is chosen from dimethylformamide, dimethyl sulfoxide and formamide.

5. Use according to any one of the previous claims, **characterized in that** the amount of EPS GY 785 polysaccharide present in the anhydrous solvent is between 1 and 10 mg/ml.

6. Use according to claim 5, **characterized by** the fact that the amount of EPS GY 785 polysaccharide present in the anhydrous solvent is between 1 and 5 mg/ml.

7. Use according to any one of the previous claims, **characterized in that** in substep a), the dissolution of the EPS GY 785 polysaccharide in the anhydrous solvent is carried out, with stirring, at room temperature for 1 to 2 hours and then at a temperature of between 40 and 50°C, for 2 hours under argon with molecular sieve.

8. Use according to any one of the previous claims, **characterized in that** the at least one chemical sulfation agent used in substep b) is chosen from complexes of pyridine sulfate, of triethylamine sulfate and of trimethylamine sulfate.

9. Use according to claim 8, **characterized in that** in substep b), the at least one chemical sulfation agent is added to the solution of EPS GY 785 polysaccharide in a weight amount representing from 4 to 6 times the mass of the EPS GY 785 polysaccharide in solution.

10. Use according to claim 9, **characterized in that** the chemical sulfation reaction is carried out with stirring for a period of between 2 and 24 hours.

11. Use according to any one of the previous claims, **characterized in that** the chemical sulfation reaction is stopped, after cooling of the reaction mixture:
- either by addition of water in a proportion equal to 1/10 to the reaction volume and adjustment of the pH of the reaction mixture to 9 with a basifying agent;
- or by precipitation in the presence of chloride-saturated acetone or of methanol and then dissolution of the precipitate in water.

12. Use according to any one of the previous claims, **characterized in that** the second step of radical depolymerization is carried out by addition, to the reaction mixture comprising the sulfated EPS GY 785 polysaccharide in the presence of a metal catalyst, of a solution of an oxidizing agent; said addition being carried out continuously and with stirring for a period of between 30 minutes and 10 hours.

13. Use according to claim 12, **characterized in that** the reaction mixture is maintained at a pH of between 6 and 8 by by the continuous addition of a basifying agent, and at a temperature of between 30 and 70°C, throughout the radical depolymerisation reaction.

14. Use according to claim 12 or claim 13, **characterized in that** the oxidizing agent is chosen from peroxides and peracids.

15. Use according to claim 14, characteried in that the oxidizing agent is a solution of hydrogen peroxide that is added at a flow rate of between V1/1000 and V1/10 ml/minute, V1 being the volume of the reaction medium containing the sulfated polysaccharide, to which the solution of hydrogen peroxide is added.

16. Use according to claim 14, **characterized in that** the solution of oxidizing agent used in the second step is a solution of hydrogen peroxide that has a concentration of between 0.1% and 0.5% by weight and that is added to the reaction mixture at a flow rate of between V1/50 and V1/500 ml/minute.

17. Use according to any one of the previous claims, **characterized in that** in the second step consisting of depolymerisation, the sulfated EPS GY 785 polysaccharide is present in the reaction mixture at a concentration of between 2 and 10 mg/ml of reaction mixture.

18. Use according to any one of claims 12 to 17, **characterized in that** the metal catalysts that can be used in the depolymerisation step are preferably chosen from Cu⁺⁺, Fe⁺⁺⁺ and Cr⁺⁺⁺ ions and the Cr₂O₇²⁻ anion.

19. Use according to any one of claims 12 to 18, **characterized in that** the metal catalyst is present in the reaction mixture at a concentration of between 10⁻³ and 10⁻¹ M.

20. Use according to any one of the previous claims, **characterized in that** the preparation process comprises an additional step of reduction of the sulfated polysaccharide derivative obtained using a reducing agent.

21. Use according to any one of the previous claims, **characterized in that** the pharmaceutical composition is used in combination with one or more growth factors.

22. Use according to any one of claims 1 to 21, **characterized in that** the pharmaceutical composition is intended to be used in the prevention and/or the treatment of ischemia.

23. Use according to any one of claims 1 to 21, **characterized in that** the pharmaceutical composition is intended to be used for promoting revascularization and vascular modelling.

## Patentansprüche

1. Verwendung von sulfatierten Polysacharidderivaten, die eine molare Masse von weniger als oder gleich 25.000 g/mol, einen Polydispersitätsindex von weniger als 2 und einen Substitutionsgrad an Sulfatresten von weniger oder gleich 45% aufweisen, wobei die Derivate gemäß einem Herstellungsverfahren erhalten werden, das mindestens die folgenden Schritte umfasst:
- einen ersten Schritt der chemischen Sulfatierung, umfassend:
a) einen ersten Teilschritt des Lösens eines vom Meeresbakterium *Alteromonas infernus* produzierten lyophilisierten Polysaccharids GY 785 in einem nichtwässrigen Lösungsmittel und
b) einen zweiten Teilschritt der Zugabe mindestens eines chemischen Sulfatierungsmittels in einer ausreichenden Menge, um sulfatierte Polysaccharidderivate zu erhalten, die einen Substitutionsgrad an Sulfatresten von weniger oder gleich 45 Gew.-% bezogen auf das Gesamtgewicht des sulfatierten Polysaccharids GY 785 aufweisen, bei einer Temperatur zwischen 45 und 60°C;
- einen zweiten Schritt der Radikaldepolymerisation des im vorangehenden Schritt erhaltenen sulfatierten Polysaccharids, um ein sulfatiertes Polysaccharid zu erhalten, das eine molare Masse von weniger als oder gleich 25.000 g/mol und einen Polydispersitätsindex von weniger als 2 aufweist,
für die Herstellung eines Arzneimittels, das für eine pro-angiogene Wirkung in der cardiovaskulären Therapie bestimmt ist.

2. Verwendung nach Anspruch 1, **dadurch gekennzeichnet, dass** im ersten Schritt das lyophylisierte EPS GY 785 in seiner nativen Form oder in Form eines Basenadditionssalzes verwendet wird.

3. Verwendung nach Anspruch 2, **dadurch gekennzeichnet, dass** das lyophilisierte EPS GY 785 in Form eines Basenadditionssalzes mit einer starken oder schwachen Base ausgewählt aus Pyridin, Triethylamin, Tributylamin, Tetrabutylammoniumhydroxid oder Natriumhydrogencarbonat verwendet wird.

4. Verwendung nach einem der vorangehenden Ansprüche, **dadurch gekennzeichnet, dass** das nichtwässrige Lösungsmittel ausgewählt ist aus Dimethylformamid, Dimethylsulfoxid und Formamid.

5. Verwendung nach einem der vorangehenden Ansprüche, **dadurch gekennzeichnet, dass** die im nichtwässrigen Lösungsmittel vorhandene Menge an EPS GY 785 zwischen 1 und 10 mg/ml liegt.

6. Verwendung nach Anspruch 5, **dadurch gekennzeichnet, dass** die im nichtwässrigen Lösungsmittel vorhandene Menge an EPS GY 785 zwischen 1 und 5 mg/ml liegt.

7. Verwendung nach einem der vorangehenden Ansprüche, **dadurch gekennzeichnet, dass** das Lösen des EPS GY 785 im nichtwässrigen Lösungsmittel in Teilschritt a) unter Schütteln bei Raumtemperatur während 1 bis 2 Stunden, dann bei einer Temperatur zwischen 40 und 50°C während 2 Stunden unter Argon unter Verwendung eines Molekularsiebs stattfindet.

8. Verwendung nach einem der vorangehenden Ansprüche, **dadurch gekennzeichnet, dass** das oder die in Teilschritt b) verwendete(n) chemische(n) Sulfatierungsmittel aus den Pyridinsulfat-, Triethylaminsulfat- und Trimethylaminsulfat-Komplexen ausgewählt ist/sind.

9. Verwendung nach einem der vorangehenden Ansprüche, **dadurch gekennzeichnet, dass** in Teilschritt b) das oder die chemische(n) Sulfatierungsmittel der EPS GY 785-Lösung in einer Gewichtsmenge zugefügt wird/werden, die der 4- bis 6-fachen Menge an EPS GY 785 in Lösung entspricht.

10. Verwendung nach einem der vorangehenden Ansprüche, **dadurch gekennzeichnet, dass** die chemische Sulfatierungsreaktion unter Schütteln während eines Zeitraums von 2 bis 24 Stunden durchgeführt wird.

11. Verwendung nach einem der vorangehenden Ansprüche, **dadurch gekennzeichnet, dass** die chemische Sulfatierungsreaktion nach dem Abkühlen des Reaktionsmediums gestoppt wird:
- entweder durch Zugabe von Wasser in einem Verhältnis von 1/10 zum Reaktionsvolumen und Einstellen des pH-Werts des Reaktionsmediums auf 9 mithilfe eines alkalisierenden Mittels;
- oder durch Ausfällen in Gegenwart von mit Natriumchlorid gesättigtem Aceton oder Methanol und anschließender Auflösung des Niederschlags in Wasser.

12. Verwendung nach einem der vorangehenden Ansprüche, **dadurch gekennzeichnet, dass** der zweite Schritt der Radikaldepolymerisation durch Zugabe einer Lösung eines Oxidationsmittels zum EPS GY 785 enthaltenden Reaktionsgemisch in Gegenwart eines Metallkatalysators; wobei die Zugabe kontinuierlich und unter Schütteln über einen Zeitraum von 30 Minuten bis 10 Stunden erfolgt.

13. Verwendung nach Anspruch 12, **dadurch gekennzeichnet, dass** das Reaktionsgemisch während der Gesamtdauer der Radikaldepolymerisationsreaktion durch kontinuierliche Zugabe eines alkalisierenden Mittels bei einem pH-Wert zwischen 6 und 8 und auf einer Temperatur zwischen 30 und 70°C gehalten wird.

14. Verwendung nach Anspruch 12 oder nach Anspruch 13, **dadurch gekennzeichnet, dass** das Oxidationsmittel aus Peroxiden und Persäuren ausgewählt ist.

15. Verwendung nach Anspruch 14, **dadurch gekennzeichnet, dass** das Oxidationsmittel eine Wasserstoffperoxidlösung ist, die in einer Menge von V1/1.000 und V1/10 ml/Minute hinzugefügt wird, wobei V1 das Volumen des Reaktionsmediums ist, das das sulfatierte Derivat von EPS GY 785 enthält und zu dem die Wasserstoffperoxidlösung hinzugefügt ist.

16. Verwendung nach Anspruch 14, **dadurch gekennzeichnet, dass** die im zweiten Schritt verwendete Lösung des Oxidationsmittels eine Wasserstoffperoxidlösung ist, die eine Konzentration zwischen 0,1 Gew.-% und 0,5 Gew.-% aufweist und die dem Reaktionsgemisch in einer Menge zwischen V1/50 und V1/500 ml/Minute hinzugefügt wird.

17. Verwendung nach einem der vorangehenden Ansprüche, **dadurch gekennzeichnet, dass** im zweiten Schritt der Depolymerisation sulfatiertes EPS GY 785 im Reaktionsgemisch in einer Konzentration von 2 bis 10 mg/ml des Reaktionsgemischs vorliegt.

18. Verwendung nach einem der Ansprüche 12 bis 17, **dadurch gekennzeichnet, dass** die Metallkatalysatoren, die im Depolymerisationsschritt verwendet werden können, bevorzugt aus den Ionen Cu⁺⁺, Fe⁺⁺, Cr⁺⁺⁺ und dem Anion Cr₂O₇⁻² ausgewählt werden.

19. Verwendung nach einem der Ansprüche 12 bis 18, **dadurch gekennzeichnet, dass** der Metallkatalysator im Reaktionsgemisch in einer Konzentration zwischen 10⁻³ M und 10⁻¹ M vorliegt.

20. Verwendung nach einem der vorangehenden Ansprüche, **dadurch gekennzeichnet, dass** das Herstellungsverfahren einen zusätzlichen Schritt der Reduktion der erhaltenen Polysaccharidderivate mithilfe eines Reduktionsmittels umfasst.

21. Verwendung nach einem der vorangehenden Ansprüche, **dadurch gekennzeichnet, dass** das Arzneimittel in Verbindung mit einem oder mehreren Wachstumsfaktoren verwendet wird.

22. Verwendung nach einem der Ansprüche 1 bis 21, **dadurch gekennzeichnet, dass** das Arzneimittel zur Vorbeugung und/oder Behandlung einer Ischämie bestimmt ist.

23. Verwendung nach einem der Ansprüche 1 bis 21, **dadurch gekennzeichnet, dass** das Arzneimittel dazu bestimmt ist, die Revaskularisation und vaskuläre Umgestaltung zu fördern.
